# EUROPEAN PATENT APPLICATION

(11) **EP 2 777 589 A1**
(43) Date of publication of application: **17.09.2014**
(21) Application number: 14159628.8
(22) Date of filing: 13.03.2014
(51) Int. Cl.: A61B 18/14

(54) **Catheter with spray irrigation**

(30) Priority: 14.03.2013 US 201313804786
(71) Applicant: Biosense Webster (Israel), Ltd., Yokneam 20692 (IL)
(72) Inventor: Govari, Assaf, 34400 Haifa (IL); Nakagawa, Hiroshi, Edmond, OK Oklahoma 73012 (US); Beeckler, Christopher Thomas, Brea, CA California 92821 (US)
(74) Representative: Carpmaels & Ransford LLP

(57) **Abstract**

A medical probe, including a flexible insertion tube having a distal end for insertion into a body cavity, and a channel contained within the insertion tube and configured to convey a fluid to the distal end. A terminal member is fixed to the distal end of the insertion tube and includes a distal tip configured to be brought into contact with tissue in the body cavity, the distal tip having a first diameter, and a protruding shoulder surrounding the distal tip and having a second diameter, which is greater than the first diameter. The distal tip has one or more spray ports passing through the shoulder which are coupled to the channel so as to direct the fluid from the shoulder toward the tissue so as to irrigate the tissue.

## Description

### FIELD OF THE INVENTION

The present invention relates generally to invasive probes, and specifically to an invasive probe configured to irrigate tissue during a medical procedure.

### BACKGROUND

A wide range of medical procedures involve placing objects such as sensors, tubes, catheters, dispensing devices, and implants, within the body. An example of a medical procedure a performed with a catheter is ablation of body tissue such as heart tissue. The ablation may be used to cure a variety of cardiac arrhythmia, as well as to manage atrial fibrillation. Such procedures are known in the art. Other medical procedures using ablation of body tissue, such as treating varicose veins, are also known in the art. The ablation energy for these procedures may be in the form of radio-frequency (RF) energy, which is supplied to the tissue via one or more electrodes of a catheter used for the procedures.

The application of the ablation energy to body tissue, if uncontrolled, may lead to an unwanted increase of temperature of the tissue. It is consequently important to monitor and control the temperature of the tissue during any medical procedure involving ablation. One method for control is to irrigate the tissue being ablated.

### SUMMARY OF THE INVENTION

There is provided, in accordance with an embodiment of the present invention a medical probe, including a flexible insertion tube having a distal end for insertion into a body cavity, a channel contained within the insertion tube and configured to convey a fluid to the distal end, and a terminal member fixed to the distal end of the insertion tube and including a distal tip configured to be brought into contact with tissue in the body cavity, the distal tip having a first diameter, and a protruding shoulder surrounding the distal tip and having a second diameter, which is greater than the first diameter, and one or more spray ports passing through the shoulder and coupled to the channel so as to direct the fluid from the shoulder toward the tissue so as to irrigate the tissue.

In some embodiments, the distal tip may include a metal tip, and the metal tip may include a temperature sensor such as a thermocouple. In alternative embodiments, the one or more spray ports may be dimensioned and oriented so to direct the fluid away from the distal tip, and a dimension of the one or more spray ports may include a spray port diameter of less than 30 µm. In further embodiments, the one or more spray ports can be configured to emit the fluid so as to create a turbulent flow in a body fluid surrounding the distal tip, wherein the turbulent flow may form one or more vortices in the body fluid. In additional embodiments, the fluid may include a saline solution, and the body fluid may include blood.

There is also provided, in accordance with an embodiment of the present invention, a method, including inserting a distal end of a flexible insertion tube into a body cavity, the insertion tube containing a channel configured to convey a fluid to the distal end, the distal end including a terminal member fixed to the distal end of the insertion tube and including a distal tip configured to be brought into contact with tissue in a body cavity, the distal tip having a first diameter and a protruding shoulder surrounding the distal tip and having a second diameter, which is greater than the first diameter. The method further includes directing, via one or more spray ports passing through the shoulder, the fluid from the shoulder toward the tissue so as to irrigate the tissue.

There is further provided, in accordance with an embodiment of the present invention, a method, including inserting a distal end of a medical probe into a body cavity containing a body fluid, and directing a flow of an irrigation fluid from the distal end of the medical probe toward a tissue in the body cavity via one or more spray ports in the distal end, wherein a pressure of the irrigation fluid and a size of the one or more spray ports are chosen so that the flow of the irrigation fluid creates a turbulent flow in the body fluid within the body cavity.

### BRIEF DESCRIPTION OF THE DRAWINGS

The disclosure is herein described, by way of example only, with reference to the accompanying drawings, wherein:
Figure 1 is a schematic, pictorial illustration of a medical system implementing spray irrigation, in accordance with an embodiment of the present invention;
Figure 2A is a schematic cross-sectional longitudinal view of a distal end of a medical probe used in the system, in accordance with an embodiment of the present invention;
Figure 2B is a schematic cross-sectional latitudinal view of the distal end of the medical probe, in accordance with an embodiment of the present invention; and
Figure 3 is a schematic detail view showing the medical probe irrigating endocardial tissue, in accordance with an embodiment of the present invention.

### DETAILED DESCRIPTION OF EMBODIMENTS

### OVERVIEW

Various therapeutic procedures such as cardiac ablation use an invasive medical probe such as a catheter that is inserted into a patient's body. During an ablation procedure on a heart, there may be local overheating of the heart surface being ablated, as well as of the heart tissue underlying the surface. The surface overheating may be manifested as charring, and the overheating of the underlying tissue may cause other damage to the tissue, even leading to penetration of the tissue. To monitor and control the temperature of the surface and the underlying tissue, as well as to estimate the temperature of the tissue, a temperature sensor may be positioned within a distal tip of the catheter. Additionally, the region being ablated may be irrigated with an irrigation fluid, typically saline, in order to prevent charring.

In addition to the risk of charring, overheating of blood in the region being ablated may cause the formation of potentially dangerous blood clots, which can grow and potentially cause a heart attack or a stroke. While the irrigation may slightly reduce blood clot formation by cooling and diluting the blood, there is still a possibility of clotting.

In embodiments of the present invention, a temperature sensor such as a thermocouple is positioned within a distal tip of a medical probe such as a catheter, and the distal end of the catheter is fitted with spray ports for irrigating tissue in the region being ablated. As explained in detail hereinbelow, the spray ports are dimensioned and oriented so that the exiting fluid, also herein termed irrigation fluid, sprays away from the distal tip. Directing the spray of the exiting fluid away from the distal tip can increase the accuracy of temperature measurements conveyed by the sensor, since the distal tip (and thus the temperature sensor within the distal tip) may not be cooled by the fluid.

In some embodiments, the spray ports are configured to spray the exiting fluid as fluid jets. Under sufficient pressure, the fluid jets form turbulent flow in body fluid, typically comprising blood possibly mixed with the exiting fluid, surrounding the distal tip. In some embodiments, the turbulent flow may appear as one or more vortices in the blood, which can help prevent formation of blood clots in the region being ablated. Therefore, medical probes incorporating the embodiments described herein can enable therapeutic procedures such as cardiac ablation to be performed with greater accuracy and patient safety.

### SYSTEM DESCRIPTION

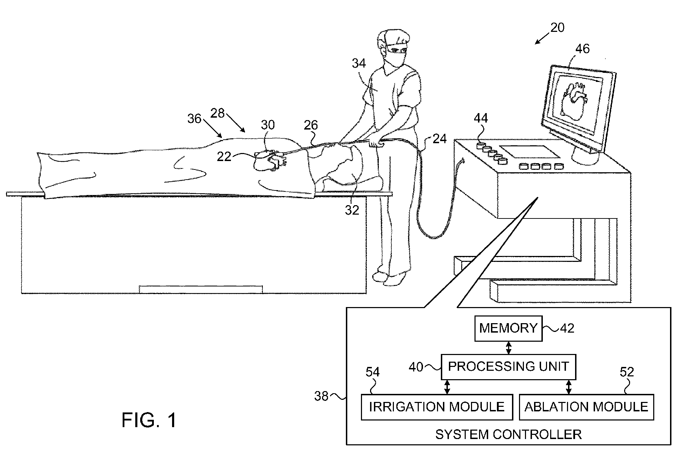

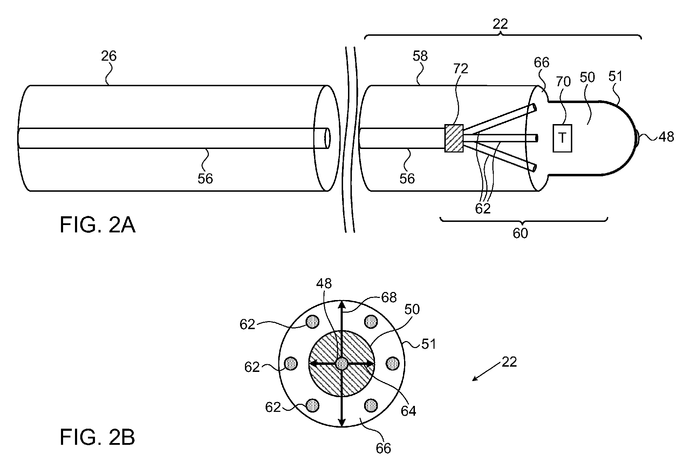

Reference is now made to Figure 1, which is a schematic, pictorial illustration of a medical system 20 implementing spray irrigation, Figure 2A which is a schematic cross-sectional longitudinal view of a distal end 22 of a medical probe 24 used in the system, and Figure 2B which is a schematic cross-sectional latitudinal view of the distal end, in accordance with an embodiment of the present. In the embodiments described herein, it is assumed that probe 24 is used for a therapeutic treatment, such performing ablation of heart tissue. Alternatively, probe 24 may be used, *mutatis mutandis,* for other therapeutic and/or diagnostic purposes in the heart or in other body organs.

In system 20, probe 24 comprises an insertion tube 26, which is inserted into a lumen 28, such as a chamber of a heart 30, of a subject 32. The probe is used by an operator 34 of system 20, during a procedure which typically includes performing ablation of body tissue 36.

For intracardiac operation, insertion tube 26 and distal end 22 should generally have a very small outer diameter, typically of the order of 2-3 mm. Therefore, all of the internal components of medical probe 24 are also made as small and as thin as possible, and are arranged so as to avoid, as much as possible, damage due to small mechanical strains.

The functioning of system 20 is managed by a system controller 38, comprising a processing unit 40 communicating with a memory 42, wherein is stored software for operation of system 20. Controller 38 is typically an industry-standard personal computer comprising a general-purpose computer processing unit. However, in some embodiments, at least some of the functions of the controller are performed using custom-designed hardware and software, such as an application specific integrated circuit (ASIC) or a field programmable gate array (FPGA). Controller 38 is typically managed by operator 34 using a pointing device 44 and a graphic user interface (GUI) displayed on a screen 46, which enable the operator to set parameters of system 20. Screen 46 typically also displays results of the procedure to the operator.

The software in memory 42 may be downloaded to the controller in electronic form, over a network, for example. Alternatively or additionally, the software may be provided on non-transitory tangible media, such as optical, magnetic, or electronic storage media.

In the example shown in Figure 2A, an electrode 48 is mounted on a distal tip 50 of medical probe 24. The electrode typically comprises a thin metal layer formed over distal tip 50. In some embodiments, distal end 22 may have other electrodes that are insulated from each other and from electrode 48, which for simplicity are not shown in the diagram. Electrode 48 is connected to system controller 38 by conductors in tube 26, not shown in the figures. As described below, electrode 48 can be used to ablate tissue 36.

System controller 38 (Fig. 1) comprises an RF ablation module 52 and an irrigation module 54. Processing unit 40 uses the ablation module to monitor and control ablation parameters such as the level of ablation power applied via electrode 48. The ablation module may also monitor and control the duration of the ablation that is provided.

Typically, during ablation, heat is generated in the electrode (or electrodes) providing the ablation, as well as in the surrounding region. In order to dissipate the heat and to improve the efficiency of the ablation process, system 20 supplies irrigation fluid to distal end 22 via a channel 56. System 20 uses irrigation module 54 to monitor and control irrigation parameters, such as the pressure and the temperature of the irrigation fluid, as is described in more detail below.

Distal end 22 is covered by a flexible, insulating sheath 58, and comprises a terminal member 60 fixed to distal end 22. When catheter probe 24 is used, for example, in ablating endocardial tissue by delivering RF electrical energy through electrode 48, considerable heat is generated in the area of distal end 22. For this reason, it is desirable that sheath 58 comprises a heat-resistant plastic material, such as polyurethane, whose shape and elasticity are not substantially affected by exposure to the heat.

As shown in Figures 2A and 2B, channel 56 is contained within insertion tube 26 and distal end 22, and is configured to deliver an irrigation fluid to spray ports 62. Terminal member 60 comprises distal tip 50 having a first diameter 64, a protruding shoulder 66 surrounding the distal tip and having a second diameter 68, which is greater than the first diameter. Spray ports 62 pass through the shoulder and are coupled to the channel so as to direct fluid exiting from the shoulder toward the tissue in order to irrigate the tissue.

Although the configuration in Figures 2A and 2B show six spray ports 62, any suitable number of spray ports passing through shoulder 66 is considered to be within the spirit and scope of the present invention. During a medical procedure (e.g., a cardiac ablation), distal tip 50 is configured to be brought into contact with tissue in a body cavity, and spray ports 38 are dimensioned and oriented to direct the irrigation fluid toward the tissue and away from the distal tip, so as to irrigate the tissue. In some embodiments, spray ports 62 may have a diameter of less than 30 µm, and shoulder 66 and distal tip 50 may have a thickness of approximately 60 µm - approximately 100 µm. Additionally, shoulder 66 may be configured so that there is no sharp transition between sheath 58 and distal tip 50.

Distal tip 50 is typically formed as a metal tip. In some embodiments the entire metal tip comprises an electrode configured to ablate tissue 36. In an alternative embodiment, distal tip 50 is covered with an insulator 51 which in turn is overlaid by electrode 48. The tip also comprises a temperature sensor 70 such as a thermocouple positioned within the distal tip. In operation, temperature sensor 70 generates a signal indicating a temperature of the tissue in contact with the distal tip.

In some embodiments, a valve 72, which is operated by controller 38 using irrigation module 54, is placed on channel 56, allowing the controller to set and/or switch the rate of flow (and thereby the pressure) of the fluid to the spray ports. Using the valve, controller 38 may set the rate of flow to the vicinity of electrode 48 according to the function performed by the electrode. For example, if electrode 48 is being used for ablation, controller 38 may increase the flow rate through the valve compared to when the electrode is not being used for ablation. Alternatively or additionally, controller 38 may alter the flow rate according to a temperature measured by temperature sensor 70. Other sensors that the controller may use for determining the flow rate include one or more additional temperature sensors in the distal end (not shown).

The irrigation fluid is typically normal saline solution, and controller 38 (using irrigation module 54 and valve 72) controls the pressure of the fluid so that fluid jets exit the spray ports under a pressure of approximately 50 pounds per square inch (psi) in order to create a turbulent flow in body fluid, typically blood which may be mixed with the fluid exiting from the spray ports, surrounding distal tip 50 (i.e., on body tissue 36). Additionally, the fluid jets may prevent the body fluid from entering the spray ports and channel 56.

### SPRAY IRRIGATION DURING ABLATION

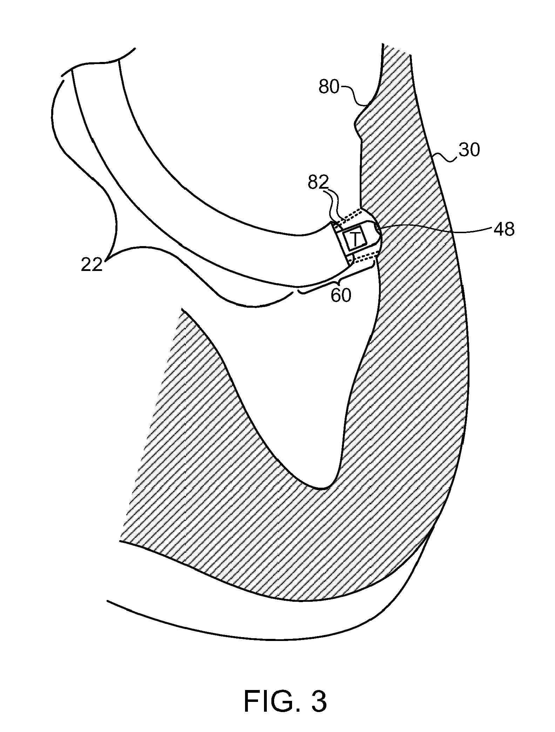

Figure 3 is a schematic detail view showing electrode 48 in contact with endocardial tissue 80 of heart 30, in accordance with an embodiment of the present invention. As described supra, during some electrophysiological therapeutic procedures, such as cardiac ablation, it is typically important to accurately monitor and regulate the temperature of the endocardial tissue.

During an ablation procedure, medical probe 24 can irrigate endocardial tissue 80 with fluid jets 82, exiting from respective ports 62, in order to cool the endocardial tissue and reduce charring. As described hereinabove, due to the configuration of spray ports 62, medical probe 20 directs jets 82 toward the endocardial tissue and away from distal tip 50. Directing jets 82 away from distal tip 30 increases the accuracy of temperatures measured by temperature sensor 70, since any contact between the irrigation fluid and the distal tip can cool the distal tip, resulting in the temperature sensor conveying a signal indicating a lower temperature (i.e., lower than actual) of the endocardial tissue. Therefore, by enabling temperature sensor 70 to accurately measure the temperature of the endocardial tissue, embodiments of the present invention reduce the risk of charring the tissue during the ablation. Additionally, as described supra, the vortices created by fluid jets 82 help prevent blood from coagulating and/or clotting in endocardial tissue 80.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

Aspects of the invention not yet claimed:
Aspect 1. A method, comprising:
   inserting a distal end of a flexible insertion tube into a body cavity, the insertion tube containing a channel configured to convey a fluid to the distal end, the distal end comprising a terminal member fixed to the distal end of the insertion tube and comprising a distal tip configured to be brought into contact with tissue in a body cavity, the distal tip having a first diameter and a protruding shoulder surrounding the distal tip and having a second diameter, which is greater than the first diameter; and
   directing, via one or more spray ports passing through the shoulder, the fluid from the shoulder toward the tissue so as to irrigate the tissue.
Aspect 2. The method according to aspect 1, wherein the distal tip comprises a metal tip.
Aspect 3. The method according to aspect 1, wherein the one or more spray ports are dimensioned and oriented so to direct the fluid away from the distal tip.
Aspect 4. The method according to aspect 3, wherein a dimension of the one or more spray ports comprises a spray port diameter of less than 30 µm.
Aspect 5. The method according to aspect 1, wherein the one or more spray ports are configured to emit the irrigation fluid so as to create a turbulent flow in a body fluid surrounding the distal tip.
Aspect 6. The method according to aspect 5, wherein the turbulent flow forms one or more vortices in the body fluid.
Aspect 7. The method according to aspect 5, wherein the fluid comprises a saline solution and wherein the body fluid comprises blood.
Aspect 8. A method, comprising:
   inserting a distal end of a medical probe into a body cavity containing a body fluid; and
   directing a flow of an irrigation fluid from the distal end of the medical probe toward a tissue in the body cavity via one or more spray ports in the distal end, wherein a pressure of the irrigation fluid and a size of the one or more spray ports are chosen so that the flow of the irrigation fluid creates a turbulent flow in the body fluid within the body cavity.
Aspect 9. The method according to aspect 8, wherein the irrigation fluid comprises a saline solution and the body fluid comprises blood.
Aspect 10. The method according to aspect 8, wherein the size comprises a diameter less than 30 µm.
Aspect 11. The method according to aspect 8, wherein the turbulent flow forms one or more vortices in the body fluid.

## Claims

1. A medical probe, comprising:
a flexible insertion tube having a distal end for insertion into a body cavity;
a channel contained within the insertion tube and configured to convey a fluid to the distal end; and
a terminal member fixed to the distal end of the insertion tube and comprising:
a distal tip configured to be brought into contact with tissue in the body cavity, the distal tip having a first diameter; and
a protruding shoulder surrounding the distal tip and having a second diameter, which is greater than the first diameter; and
one or more spray ports passing through the shoulder and coupled to the channel so as to direct the fluid from the shoulder toward the tissue so as to irrigate the tissue.

2. The probe according to claim 1, wherein the distal tip comprises a metal tip.

3. The probe according to claim 1, and comprising a temperature sensor contained within the distal tip.

4. The probe according to claim 3, wherein the temperature sensor comprises a thermocouple.

5. The probe according to claim 1, wherein the one or more spray ports are dimensioned and oriented so to direct the fluid away from the distal tip.

6. The probe according to claim 5, wherein a dimension of the one or more spray ports comprises a spray port diameter of less than 30 µm.

7. The probe according to claim 1, wherein the one or more spray ports are configured to emit the fluid so as to create a turbulent flow in a body fluid surrounding the distal tip.

8. The probe according to claim 7, wherein the turbulent flow forms one or more vortices in the body fluid.

9. The probe according to claim 7, wherein the fluid comprises a saline solution, and wherein the body fluid comprises blood.
